Europäisches Patentamt

European Patent Office

Office européen des brevets

(11) Publication number:
**0 293 193**
**A2**

# (12) EUROPEAN PATENT APPLICATION

(21) Application number: **88304759.9**

(22) Date of filing: **26.05.88**

(51) Int. Cl.⁴: **C 12 N 15/00**
**C 12 N 7/00, A 61 K 35/76**

(30) Priority: **28.05.87 GB 8712528**

(43) Date of publication of application:
**30.11.88 Bulletin 88/48**

(84) Designated Contracting States: **ES GR**

(71) Applicant: **Clayton Foundation for Research**
**Three Riverway Suite 1625**
**Houston Texas 77056 (US)**

(72) Inventor: **Howell, Stephen Barnard**
**13612 Nogales Drive**
**Del Mar California 92014 (US)**

**Verma, Inder**
**448 Marview Drive**
**Solora Beach California 92075 (US)**

(74) Representative: **Wilkinson, Stephen John et al**
**c/o Stevens, Hewlett & Perkins 5 Quality Court Chancery Lane**
**London WC2A 1HZ (GB)**

(54) Gene transfer for drug resistance.

(57) A method for sensitizing mammalian tumor cells which comprises the step of inserting a drug sensitivity gene into the tumor cell. Also disclosed is the use of hypoxanthine phosphoribosyltransferase for the treatment of leukemia when the drug 6-thioguanine is used. The method employs a composition of matter which includes a 3' long terminal repeat and a 5' long terminal repeat, each repeat containing a restriction enzyme site; between the repeats is inserted a promoter and/or enhancer gene and the drug sensitivity gene. All of the elements of the DNA are linked and spatially positioned, such that, when the gene is inserted into a target cell the drug sensitivity gene is expressed.

EP 0 293 193 A2

**Description**

## GENE TRANSFER FOR DRUG RESISTANCE

### Field of Invention

The present invention relates generally to the insertion of drug sensitivity genes into mammalian tumor tissues which have lost their sensitivity to anti-neoplastic drugs. More particularly it relates to the insertion of anabolic enzymes whose function has been lost in the tumor cell population. The insertion is achieved using retroviral vectors.

### Background of the Invention

The development of drug resistance is a common problem in the treatment of malignancies. Unfortunately, cells which are initially sensitive to anti-cancer drugs, frequently acquire drug resistance. This severely limits the success of chemotherapy. In some cell types, resistance can be attributed to the loss of an enzyme necessary for activation of the anti-neoplastic drug. Genes which code for critical anabolic enzymes are in effect "drug sensitivity" genes. Mutational loss of the function of these genes potentially can be corrected by gene transfer. By inserting the replacement gene into the tumor cell population, it may be possible to restore anti-neoplastic drug sensitivity.

Replication-defective retroviral vectors have been used in attempts to introduce new genetic material into mammalian cells. Specifically, this approach has been used to transfer the human hypoxanthine-guanine phosphoribosyltransferase (HGPRT) gene into murine and human bone marrow stem cells and lymphocytes. This work has been directed to somatic cell gene therapy for inherited diseases.

There are a number of advantages in using retroviral vectors for the transfer of genetic information into human cells. Since the process of gene transfer is very efficient, theoretically, nearly all of the drug resistant cells could be transformed to a drug sensitive state. Secondly, the foreign gene is inserted with a defined structure. Finally, the inserted genes should be sufficiently stable after integration into the cells to permit killing tumor cells by the subsequent administration of appropriate chemotherapeutic agents.

6-thioguanine is a drug extensively used in the treatment of human leukemia. Before 6-thioguanine can exert its cytotoxic effect it must be converted by HGPRT to 6-thioquanosine monophosphate. Approximately 7% of human leukemias resistant to thiopurines have a severe deficiency of HGPRT. An additional 14% of all resistant cases show partial loss of HGPRT activity. In experimental tumors, the marked loss of HGPRT activity has been the most common cause of thiopurine insensitivity.

Approximately 50% of the children with acute lymphoblastic leukemia relapse following chemotherapy with purine antimetabilites, and in patients with acute non-lymphocytic leukemia the relapse rate is even higher. One of the causes of clinical 6-thioguanine resistance in patients is a decrease in HGPRT activity. The extent to which loss of HGPRT activity is the primary cause of drug resistance in these patients has not been well defined.

The present invention provides a model for overcoming drug resistance in the treatment of human malignancies by providing a method for the insertion of drug sensitivity genes into tumor cells. HGPRT gene therapy can be viewed as one potential means of overcoming clinical 6-thioguanine resistance mediated by the loss of HGPRT activity in the cells of leukemia patients.

### Summary of the Invention

An object of the present invention is a method for sensitizing tumor cells.

An additional object of the present invention is a method of treating malignant cells.

A further object of the present invention is the development of a retrovirus vector for inserting a drug sensitivity gene into mammalian cells.

Another object of the present invention is the treatment of leukemia.

Thus, in accomplishing the foregoing objects there is provided in accordance with one aspect of the present invention, a method of sensitizing a tumor cell comprising the step of inserting a drug sensitivity gene into the tumor cell. One aspect of the present invention is the insertion of the gene for hypoxanthine-guanine phosphoribosyltransferase into a tumor cell. In the preferred embodiment the insertion step is repeated at least once.

Another aspect of the present invention is a method of treating a malignant cell comprising the steps of inserting a drug sensitivity gene into said malignant cell by exposing the malignant cell to a retrovirus which includes the drug sensitivity gene; and exposing the resulting cell to an anti-cancer drug. In one preferred embodiment a human leukemic cell is treated by the insertion of the hypoxanthine-guanine phosphoribosyltransferase gene and treatment with 6-thioguanine.

A further aspect of the present invention is as a composition of matter, a retrovirus, comprising DNA segments including a 3′ long terminal repeat, and a 5′ long terminal repeat, wherein the 3′ and 5′ long terminal repeats each contain at least one restriction enzyme site and are attached to the 3′ and 5′ ends respectively; a promoter/enhancer; and at least one drug sensitivity gene, wherein the DNA segments are linked together and spatially positioned to express the at least one drug sensitivity gene after insertion into a target cell. In a preferred embodiment the composition of matter includes a mouse short metallothionein promoter and the hypoxanthine-guanine phosphoribosyltransferase gene.

Further objects, features and advantages will be apparent in the following description of the preferred embodiments of the invention.

### Brief Description of the Drawings

Figure 1A shows a map of the general structure of a retrovirus for the insertion of a drug sensitivity gene into mammalian cells.

Figure 1B shows a map of the HGPRT provirus LPHAL. The scale is in kilobase pairs.

Figure 2 shows the percent of HGPRT negative cells sensitized to 6-thioguanine as a function of exposure time to the LPHAL provirus.

Figure 3 shows the effect of viral titer on increasing HGPRT activity following exposure to LPHAL.

Figure 4 shows the effect of the time between viral exposure and the start of drug selection on the fraction of HGPRT negative cells sensitized to 6-thioguanine.

Figure 5 is a histogram of HGPRT activity present in 40 clones of HPPRT negative cells selected in HAT medium after exposure to LPHAL.

Figure 6A shows the change in the fraction of cells sensitized with 6-thioguanine as a function of time after exposure to LPHAL.

Figure 6B shows the HGPRT activity in the whole cell population as a function of time after exposure to LPHAL.

### Detailed Description of Preferred Embodiments

It will be readily apparent to one skilled in the art that various substitutions and modifications may be made to the invention disclosed herein without departing from the scope and spirit of the invention.

The term "sensitivity gene" as used herein is a gene for an enzyme or protein which, when inserted into a tumor cell, will make that cell sensitive to chemotheraputic agents. Although the sensitivity gene may be a normal constituent of cells, it is useful because the tumor cells, to which it is inserted, usually have lost the function of the normally present gene.

As used herein the term "promoter" refers to the cite on the DNA to which RNA polymerase binds preparatory to initiating transcription of an operon. An "enhancer" as used herein refers to a modifier gene with acts to enhanced the action of a nonalleic gene.

As used herein "restriction enzyme site" refers to a sequence in DNA which is sensitive to restriction endonuclease. When the specific restriction endonuclease is added to the DNA it cleaves the DNA at that specific site. There are a variety of endonucleases which cleave different sequences. Thus, the restriction enzyme site can be tailored for each specific use

As used herein the terms "tumor cell", "malignancies" and "cancer" refer to those neoplasms and abnormal masses of cells which result from excessive cellular multiplication, growth or proliferation and which usually terminate in death if not checked by treatment.

One embodiment of the present invention is a method of sensitizing a mammalian tumor cell, comprising the step of inserting a drug sensitivity gene into said tumor cell. For example, a retrovirus containing the human HGPRT gene can be inserted into leukemic cells.

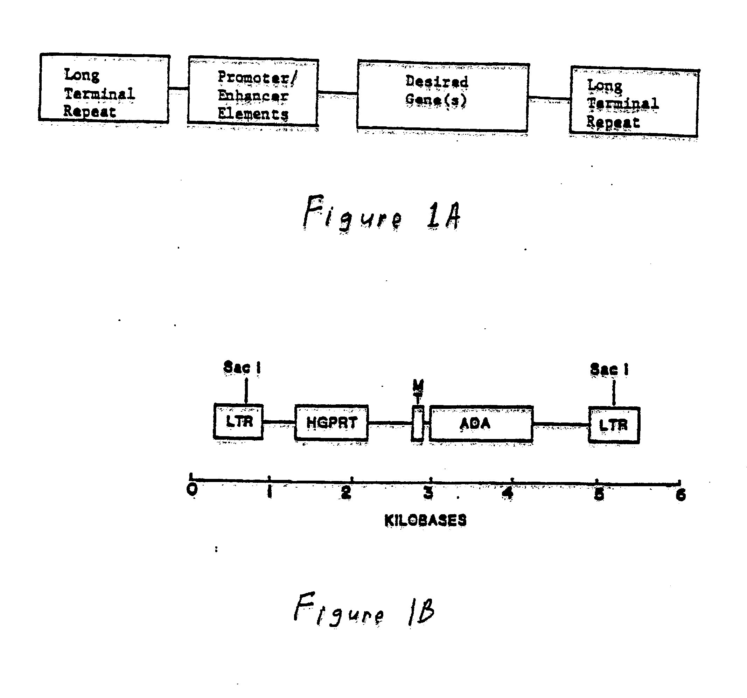

A retrovirus containing the human HGPRT and a non-expressed human adenosine deaminase gene was developed starting with the pLPMKL construct containing the human HGRPT gene coupled to the mouse short metallothionein promoter followed by the murine thymidine kinase gene. The 5' long terminal repeat is derived from the Moloney sarcoma virus, and the 3' long terminal repeat is from the Moloney leukemia virus. The long murine metallothionein promoter coupled to the human adenosine deaminase gene was cut out of the MAMD plasmid into which the adenosine deaminase gene was initially cloned. and inserted into pLPMKL at an Eco RI site to form pLPHAL. This construct contains a single SacI site in each long terminal repeat. In the construction of this plasmid two deletions took place, one in the metallothtonein promoter and the other in the region immediately upstream of the 3' long terminal repeat. The former rendered the metallothionein promoter non-functional and prevented the expression of the ADA gene in the final viral construct. A digaram of this construct is shown in figure 1A. A helper-virus free amphotrophic replication-defective virus was obtained from pLPHAL. This involved the pLPHAL being transfected into PA12 cells, and harvesting the transiently expressed virus at about 48 hours. This was used to infect B77 HGRPT negative mouse 3T3 fibroblasts. These cells were then selected in HAT medium, and several clones were co-transfected with the replication-defective packaging vector pPAM and the dihydrofolate reductase-containing plasmid FR400. The co-transfected cells were selected with 0.2μm methorexate, and the virus-producing clones obtained were tested for the "titer" of the virus they produced on rat 208 F HGPRT deficient cells in HAT medium. Virus-containing supernates were titrated and used for infection. They were prepared by splitting a confluent flask 1:5, allowing them to grow for about 24 hours, then changing the medium, and harvesting the supernate at about 24 hours. The supernate was centrifuged a 1000 x g for 10 minutes to pellet the unattached cells. All infections are carried out in the presence of 4 μg/ml polybrene.

Figure 1B shows a general pattern of a retroviral construct which can be used for the insertion of genes into the mammalian tumor cells. In this model a long terminal repeat is found in both the 5' and 3' ends. The long terminal repeat in the preferred embodiment contains a restriction site. In addition to containing the long terminal repeats the retrovirus also contains promoter/enhancer elements. At this location a promoter gene and/or an enhancer gene can be inserted to facilitate the expression of the desired genes in the mammalian cells. The desired genes in the present invention include at least one drug sensitivity gene. A variety of promoter gene combinations can be inserted into the retroviral vector for the expression of multiple sensitivity genes within a cell. The DNA segments from the long terminal repeats and promoter/enhancer and drug sensitivity genes must be properly oriented. The DNA fragments when linked must be spatially positioned when the retrovirus is inserted into a mammalian cell. When the elements are positioned correctly the drug sensitivity gene is expressed.

In the preferred embodiment the promoter is the mouse short matallothionein promoter and the drug sensitivity gene is the HGPRT gene, and the restriction site is the Sac I site. The target cell is any tumor, cancer or malignant cell which has lost sensitivity to chemotheraphy. Insertion of the gene returns sensitivity and the cells are killed by chemotherapeutic agents

The retrovirus construction involves linking cloned DNA segments together extracellularly, transfecting the DNA construct into an eukaryotic cell, harvesting the virus produced, and using this virus to infect the target malignant cell. Preparation of the virus may involve several transfection and infection steps to give the final virus the desired characteristics of being able to efficiently infect malignant human cells and express the desired gene(s) at high level without being able to replicate extensively.

A variety of genes can be transferable or potentially transferable by this technique to sensitize cells, including genes that code for sensitivity to anti-cancer drugs, genes that suppress the malignant phenotype of human tumors, bacterial genes that code for the conversion of an antibotic to a toxic form capable of killing the malignant cell, genes coding for the production of a bacterial or fungal toxins, genes that alter the metastatic phenotype of the target cell, and genes that enhance the sensitivity to radiation

In the method of sensitizing mammalian tumor cells the drug sensitivity gene in the preferred embodiment is HGPRT. Furthermore, in the preferred embodiment it is found that performing the insertion step more than once increases the number of cells which integrate the gene and thus increases the effectiveness of the treatment.

Another embodiment of the invention includes a method of treating a mammalian malignant cell, comprising the steps of inserting the drug sensitivity gene into said malignant cell by exposing said malignant cell to a retrovirus which includes a drug sensitivity gene; and exposing the resultant cell to an anti-cancer drug. It is important that when the drug sensitivity gene is inserted, the appropriate drug which reacts with that gene is used. In the preferred embodiment, repeating the insertion and/or exposing steps more than once results in a much more efficient killing of the cancer cells and therefore a more effective treatment.

Materials and Methods

## Cells and culture.

Parental and HGPRT negative HL-60 cells were grown at RMPI 1640 supplemented with 20% heat inactivated fetal bovine serum, 1% L-glutamine and 1% antibotic mixture containing penicillin, streptomyin, amphoteracin. The LPHAL virus-producing B77 cells were grown in the same medium supplemented with only 10% fetal calf serum. Cell counts were performed with a hemocytometer in the presence of trypan blue dye.

## Clonogenic assays.

Survival of HL-60 cells was determined following virus exposure by the formation of colonies in 0.3% low melting agarose in the presence or absence of 20$\mu$M 6-thioguanine. Eighty thousand cells were plated in a final volume of 1 ml in 35 mm tissue culture dishes, then incubated in high humidity at 37° C and 5% $CO_2$ for 7-10 days. Clusters containing more than 60 cells were scored as colonies.

## Measurement of HGPRT activity by hypoxanthine uptake.

Cells to be tested for uptake of $^3$H-hypoxanthine were centrifuged and resuspended in RPMI 1640 and dialyzed heat inactivated fetal bovine serum. An aliquout of cells was removed to determine cell number. $^3$H-hypoxanthine (1 Ci/mmol, 217 pM) was added to the remaining cells and they were incubated for about 30 min at 37° C and 5% $CO_2$. Uptake was halted by the addition of 5 volumes of ice cold phosphate buffered saline. Cells were centrifuged (1000 x g), washed twice and digested overnight with 0.4 ml 0.1 N NaOH. Hypoxanthine uptake was determined by counting cell digests in a beta scintillation cocktail.

## HGPRT activity in cell lysates.

HGPRT activity of HL-60 cells was measured by slight modification of standard methods. Cells were prepared by rinsing three times with phosphate buffered saline and lysed by four cycles of freeze-thawing. Reaction mixtures for each lysate were set up in microfuge tubes on ice with 100 mM tris-HC1(pH 7.4), 10 mM $MgCl_2$, 2.0 mg/ml 5-phosphoribosyl-1-pyrophosphate. magnesium salt (PRPP) and 45 $\mu$M $^{14}$C-hypoxanthine (13.5 mCi/mmol) in a final volume of 100 $\mu$l. Cell lysates of 50 $\mu$l were added to each reaction mixture and incubated at 37° C for 15 min. Microfuge tubes were placed on ice and 50 $\mu$l of each reaction mixture was spotted onto 2.3 cm DE-81 filter discs. Filters were washed once with 1mM $NH_4HCO_3$ and 5 times with double-distilled water, then dried with methanol on a manifold device. Conversion of $^{14}$C-hypoxanthine to $^{14}$C-inosine monophosphate was determined by beta scintillation counting of dried filters.

## HAT selection.

HFPRT-positive cells HL-60 cells were selected by grown in medium containing 30 uM hypoxanthine, 0.01 uM aminopeterin, and 20 uM thymidine. All other types were selected in HAT medium containing 1 uM aminopterin.

Protein determination.

Protein content of cell lysates were determined by standard method.

Southern blots.

DNA was digested with Sac I 10 μg of digested DNA from each cell line was separated by electrophoresis in 1% agarose and transferred to nitrocellulose by standard methods described in Southern 98 Mol. Biol. 503-517, (1975). Filters were probed with a 0.6 kb nick-transplated fragment of the human HGPRT cut out of pLPL with PstI and HindIII. Hybridization was performed in 6 x SSC (0.15 M NaCl plus 0.015 M sodium citrate, pH 7), at 42°C for 72 hours. Filters were washed twice in 2 x SSC, 0.1% sodium dodecyl sulfate for 10 minutes each at 42°C followed by two washes in 0.2 x SSC, 0.1% sodium dodecyl sulfate at 68°C for 15 minutes each.

Example 1

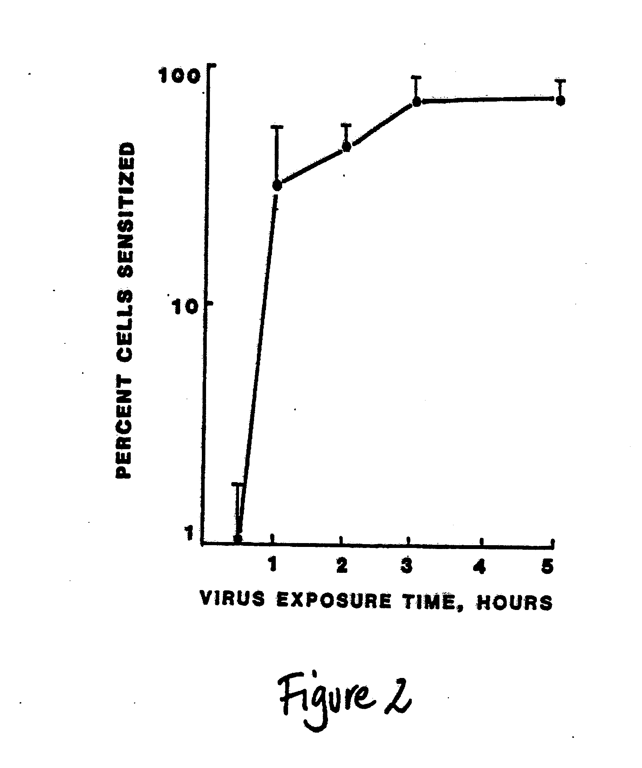

Amphotrophic replication-deficient helper-free retroviral vectors containing the human HGPRT designated LPHAL were used. The titer of viral supernates harvested from LPHAL-producing cells 18 hours after changing the medium ranged from $1.2 - 6.2 \times 10^6$ infectious viral particles/ml when tested on HGPRT negative 208 F rat cells. The efficiency of infection was assayed by suspending $10^6$ HGPRT negative HL-60 cells in 1 ml of viral supernate for 3 hours (virus:cell ratio 6.2:1), then growing them for 48 hours to allow integration and expression before they were cultured in a colony formation assay with 20 μM 6-thioguanine. A mean of $70 \pm 18\%$ (SD) of the clonogenic cells were rendered sensitive to the drug. Figure 2 shows that the fraction of cells sensitized to 6-thioguanine increased with prolongation of the duration of exposure to the viral supernate, reaching 74% at 5 hours. When HL-60 cells were co-cultivated with virus-producing cells for 48 hours, a mean of $89 \pm 3\%$ (SD) of the cells were rendered sensitive to 6-thioguanine, suggesting that continuous exposure to virus was not markedly more effective than a 5 hour exposure

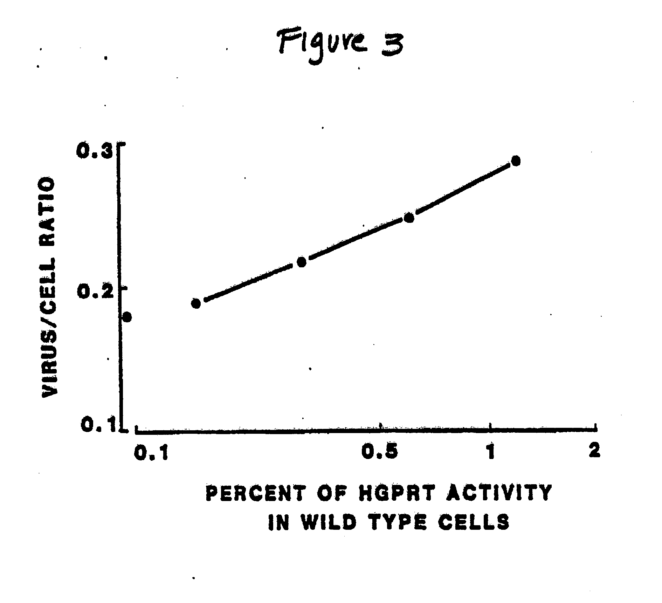

Figure 3 shows the effect of increasing the virus to cell ratio during a 3 hour infection by diluting the viral supernate. In this case the endpoint was the HGPRT activity of the infected cells measured by $^3$H-hypoxanthine uptake. The untreated HGPRT negative cells contained 0.18% of the activity present in the wild type cells. The HGPRT activity of the infected cells increased with increase in the virus:cell ratio; at a ratio of 1.2:1 the infected cells contained 60% more HGPRT activity then the uninfected cells when tested 2 days later following virus exposure

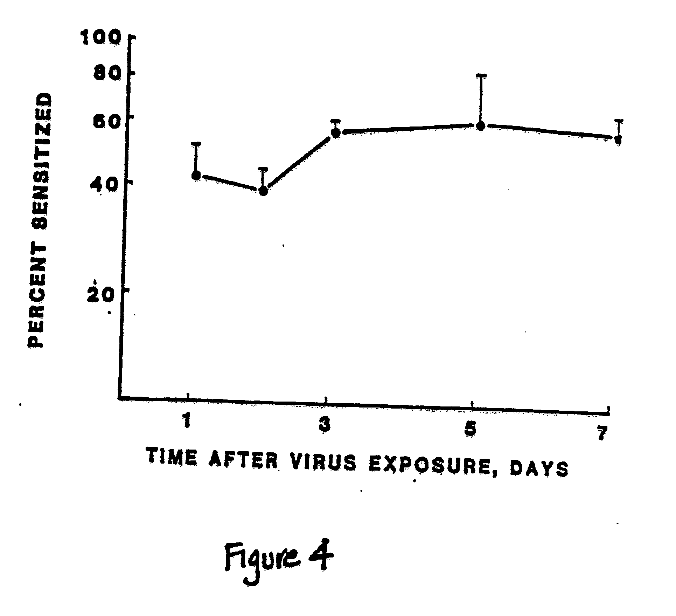

The effect of increasing the time allowed for the proviral integration and expression as measured by cell survival in a clonogenic assay is depicted in figure 4. Cells were infected for 3 hours and then cultured in drug-free media for varying lengths of time before being tested for clonogenic survival in the presence of 20 μM 6-thioguanine. The percent of clonogenic cells sensitized average 43.2% when tested 1 day after viral exposure. This percent increased to an average of 62.3% when tested 5 days after viral exposure, but the increase in the precent sensitized with time beyond 1 day was not statistically significant

The survival of clonogenic wild type HL-60 cells exposed to viral supernate at a virus:cell ratio of 1.2:1 and then treated with increasing concentrations of 6-thioguanine was no different than that of HGPRT replete HL-60 cells not exposed to virus. The ratio of the $IC_{50}$ for the parental HL-60 cells to that of the LPHAL-treated HL-60 cells was $0.6 \pm 0.3$ (SD). This suggests that the wild type HL-60 cells had an excess of HGPRT relative to that needed to sensitize them to 6-thioguanine and that any further increase contributed by the virally inserted HGPRT gene did not result in further sensitization to 6-thioguanine. Thus, in treating human tumor cells any insertion into normal cells will not affect normal cell death. The insertion of sensitivity genes will only increase the sensitivity of those cells which were previously inserted because of a lack of the enzyme necessary to respond to the anti-cancer agent.

Example II

DNA from the virus-producing murine cells, the HGPRT deficient HL-60 cells, and clones of the latter cells selected for HGPRT expression in HAT medium following viral exposure were digested with Sac I which cuts once in each proviral long terminal repeat. A Southern blot was probed with a 0.6 kb fragment from the 3' end of the human HGPRT cDNA. The LPHAL-producing murine cells contained a 5.3 kb band reflecting the presence of the HGPRT-containing provirus. The HGPRT negative HL-60 cells contained no insert, but each of the 7 HGPRT expressing clones contained the same 5.3 kb insert. Thus, cells rendered HGPRT positive by exposure to the virus each contained the integrated provirus yet had not undergone rearrangements gross enough to be detected by Southern analysis.

Example III

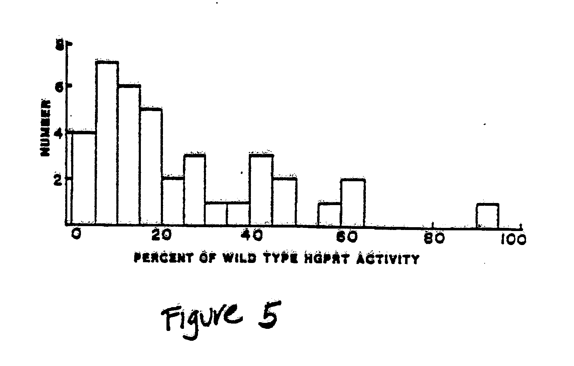

Clones of HL-60 negative cells exposed to virus and selected in HAT medium were tested for their level of HGPRT activity as measured by uptake of $^3$H-hypoxanthine. Figure 5 demonstrates that the HGPRT activity in 40 such clones ranged from 1 to 92% of the activity in wild type HL-60 HGPRT replete cells. The HGPRT activity of most of the clones was below 20.0% of the activity of wild type cells, but occasional clones were obtained with nearly normal activity levels. Figure 5 also demonstrates that even clones with as little as 5% of normal activity were capable of surviving in HAT medium.

Example IV

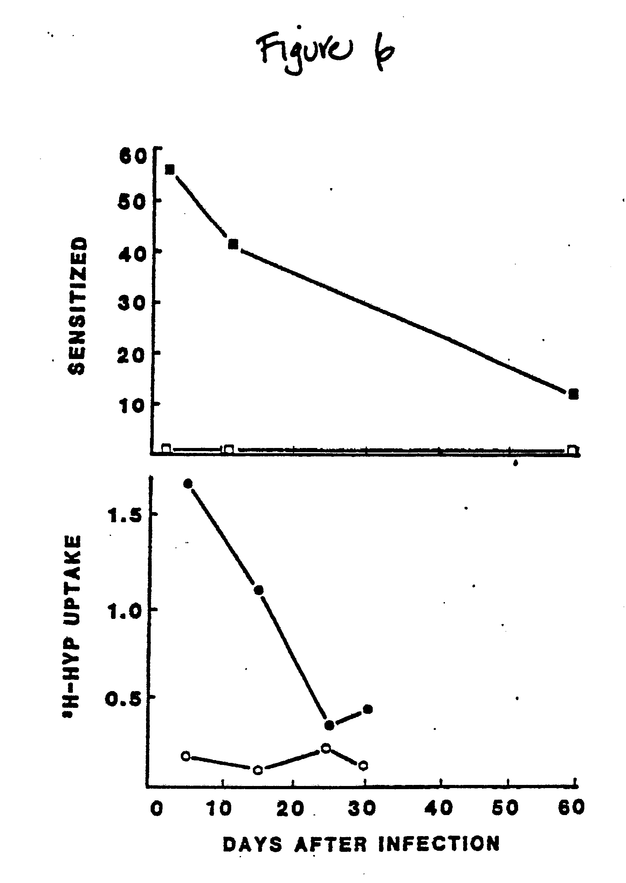

To monitor the change induced in the phenotype of the whole HGPRT negative population infection, HGPRT negative cells were tested for both their sensitivity to 6-thioguanine and their HGPRT activity following a single 3 hour exposure to virus. As shown in Figure 6a, 56% of the resistant cells had been rendered sensitive to 6-thioguanine when tested 48 hours after viral exposure. There was a slow decay in the fraction of cells remaining sensitive during subsequent growth under non-selective conditions reaching 12% at 54 days. Figure 6B shows the change in HGPRT activity as measured by the rate of accumulation of hypoxanthine in whole cells as a function of time after viral exposure. The rate of hypoxanthine uptake in HGPRT negative cells not exposed to virus was consistently less than 0.2% of that in wild type HGPRT positive cells. At 5 days after a 3 hour viral exposure, the cells accumulated hypoxanthine at 1.64% of the wild type rate; this decayed over 30 days during growth under non-selective conditions. The average growth rate of clones containing the inserted HGPRT gene was the same as the wild type HGPRT positive cells.

## Example V

Mutation rates of the inserted genes were determined using Luria-Delbruck fluctuation analysis for loss of HGPRT activity in the population of HGPRT deficient cells that had been rendered HGPRT positive by exposure to LPHAL. The forward mutation rate at the HGPRT locus in the wild type cells was $6.9 \times 10^{-8}$ per cell division. HGPRT activity coded for by the virally inserted gene was lost with a mutation rate of $1.7 \times 10^{-5}$ per cell division; a rate 241 times greater than that of the wild type cells.

## Example VI

Since a single exposure to LPHAL sensitized only a fraction of the HGPRT negative cells, successful use of gene therapy to overcome 6-thiogunanine resistance in the preferred embodiment will require that cells not rendered drug-sensitive with the first exposure be sensitized by a second or subsequent exposure. HGPRT negative HL-60 cells were exposed to LPHAL for 3 hours, and 9 clones were selected that were still resistant to 20 µM 6-thioguanine. When the Sac I digested DNA was probed with the human HGPRT cDNA, none of these clones contained the expected 5.3 kb band corresponding to the proviral HGPRT, suggesting that the reason the cells had not been sensitized was because of the failure of infection or integration rather than a failure of the expression of the integrated proviral gene. To demonstrate that these cells were still infectable, HGPRT negative HL-60 cells remaining resistant to 6-thioguanine after a single 3 hour viral exposure were retreated with virus for 3 hours, and 56 ± 4% (SD) were rendered sensitive to 6-thioguanine.

These results demonstrate the feasibility of using a retroviral vector to insert a drug-sensitivity gene into a human tumor cell. For example, in a promyelocytic leukemic tumor cell, a single 3 hour exposure of HL-60 HGPRT negative cells to the LPHAL retrovirus was sufficient to sensitize approximately 70% of the clonogenic cells. The efficiency of sensitization increased as a function of time of viral exposure and viral titer. The greatest percentage of cells was sensitized to 6-thioguanine by exposre to undiluted viral supernate. The use of an even higher virus concentration might increase the fraction of cells sensitized even further. Thus, a virus exposure of 5 hours produced 88% of the sensitization achieved by a 48 hour continuous exposure to viral-producing cells achieved through co-cultivation.

One potential problem of gene therapy as a means of restoring drug sensitivity in resistant cells is that, if the virus infects the normal cells of the host, it may increase the sensitivity of these cells as well as the resistant malignant cells limiting the therapeutic index of subsequent drug administration. However, the results presented here suggest that this is not a problem. For example, in the parental HL-60 cells, HGPRT replete cells appear to contain excess HGPRT activity above that required for maximal activation of 6-thioguanine. under these conditions, any additional HGPRT activity contributed by the virally inserted gene would not be expected to further sensitize these cells to 6-thioguanine, and in fact wild type HL-60 cells were not further sensitized by viral exposure.

Clones containing sufficient HGPRT activity to survive selection in HAT medium contained at least one copy of the proviral HGPRT genome. Likewise, clones selected for persistent lack of HGPRT activity following LPHAL exposure contained the virally inserted HGPRT. Thus, the failure of sensitization following viral exposure is probably due to failure of infection rather than failure of expression or integration of the provirus. This is of particular importance with respect to the potential use of gene therapy to reverse clinical drug resistance, since if the cells were successfully infected but did not express the drug sensitivity gene, the expectation is that they would be resistant to a subsequent infection by the same retrovirus. In fact, when cells remaining HGPRT negative after viral exposure were re-infected, we found that the efficiency of sensitization was not significantly different from that achieved with virgin HGPRT negative HL-60 cells. Hence, repeated rounds of viral infection followed by 6-thioguanine therapy can be effective in eliminating all resistant cells in a patient.

The therapeutic utility of this approach to overcoming drug resistance depends on the inserted gene remaining active long enough to permit administration of 6-thioguanine. The studies reported here clearly demonstrate the HGPRT activity attributable to the inserted gene remains in the tumor cell population for many days following viral exposure. The mutation rate of the inserted gene is, however, 241 times greater than that of the endogenous gene, which may account for the gradual loss of HGPRT activity in the whole population over a period of weeks.

In these studies we have examined the ability of LPHAL to transfer the HGPRT gene to the clonogenic cells in the population rather than to all the cells. The reason for this is that although only a small fraction of cells in a malignant population are clonogenic cells, it is these stem-like cells that are crucial to further tumor growth and are thus the most important targets for chemotherapy. In this system colony formation is the best available marker for unlimited proliferative potential. The results presented here indicate that a large fraction of the clonogenic cells can be sensitized to 6-thioguanine through insertion of the HGPRT gene. However, the results also suggest that transfer of the gene to clonogenic cells may be more efficient than transfer to non-clonogenic cells, since the HGPRT activity in the whole population after exposure of HGPRT negative cells to LPHAL remained far below the HGPRT activity present in the wild type cells.

In these studies we have used the HGPRT gene as an example of a model "drug sensitivity" gene to assess the efficacy of the retroviral vector. While it is apparent that the efficiency of infection is low enough that repeated rounds of infection and chemotherapy will be required, it is nevertheless high enough to make this approach effective for the insertion of other "drug sensitivity" genes capable of activiating chemotherapeutic drugs.

One skilled in the art will readily appreciate the present invention is well adapted to carry out the objects and obtain the ends and advantages mentioned, as well as those inherent therein. The methods, procedures and techniques described herein are presently representative of the preferred embodiments, are intended to be exemplary, and are not intended as limitations on the scope. Changes therein and other uses will occur to those skilled in the art which are encompassed within the spirit of the invention or defined by the scope of the appended claims.

**Claims**

1. A method of sensitizing a mammalian tumor cell, comprising the step of inserting a drug sensitivity gene into said tumor cell.

2. The method of claim 1, wherein said design sensitivity gene is selected from the group consisting of genes which code for sensitivity to anti-cancer drugs, genes that suppress the malignant phenotype, bacterial genes which code for the conversion of an antibiotic to a toxic form, genes coding for bacterial or fungal toxins, genes which alter the metastatic phenotype of target cells and genes that enhance sensitivity to radiation.

3. The method of claim 1 wherein said gene codes for hypoxanthine-guanine phosphoribosyltransferase.

4. The method of claim 1, wherein the inserting step is repeated at least once.

5. The method of claim 1 wherein the inserting step includes exposing said tumor cells to a retrovirus containing said drug sensitivity gene.

6. The method of treating a mammalian malignant cell, comprising the steps of:

inserting a drug sensitivity gene into said malignant cell by exposing said malignant cell to a retrovirus which includes the drug sensitivity gene; and

exposing the resultant cell to an anti-cancer drug.

7. The method of claim 6, wherein said malignant cell is exposed to retrovirus more than once.

8. The method of claim 6, wherein

said malignant cell is a human leukemic cell;

said drug sensitive gene is the hypoxanthine-guanine phosphoribosyltransferase gene; and

said anti-cancer drug is 6-thioguanine.

9. As a composition of matter, a retrovirus, comprising, DNA segments including:

a 3′ long terminal repeat, and a 5′ long terminal repeat, wherein the 3′ and 5′ long terminal repeats each contain at least one restriction enzyme site and are attached to the 3′ and 5′ ends respectively;

a promoter/enhancer; and

at least one drug sensitivity gene,

wherein the DNA segments are linked together and spatially positioned to express the at least one drug sensitivity gene after insertion into a target cell.

10. The composition of matter of claim 9, wherein

the promoter/enhancer is the mouse short metallothionein promoter; and

the at least one drug sensitivity gene codes for hypoxanthine-guanine phosphoribosyltransferase.

Figure 1A

Figure 1B

0293193

Figure 2

Figure 3

Figure 4

PERCENT OF WILD TYPE HGPRT ACTIVITY

Figure 5

Figure 6

0293193